# EUROPEAN PATENT APPLICATION

(11) **EP 2 698 171 A1**
(43) Date of publication of application: **19.02.2014**
(21) Application number: 12180382.9
(22) Date of filing: 14.08.2012
(51) Int. Cl.: A61L 2/07

(54) **Sterilisation method of dental components comprising polymethyl methacrylate**

(71) Applicant: Straumann Holding AG, 4002 Basel (CH)
(72) Inventor: van Ophuysen, Andreas, 4002 Basel (CH)
(74) Representative: Valea AB

(57) **Abstract**

A process for steam sterilisation of a dental secondary component at least partially formed from thermoset PMMA is disclosed. Also disclosed is a kit comprising a dental secondary component at least partially formed from thermoset PMMA and a tray for steam sterilisation. Further disclosed is a sterile dental secondary component at least partially formed from thermoset PMMA obtainable by the steam sterilisation process.

## Description

### Technical field

This document is directed towards dental components comprising PMMA (poly(methyl methacrylate)). More particularly, this document is directed to a process for sterilising such PMMA components using steam sterilisation. Kits for sterilisation of PMMA components and sterile PMMA components are also disclosed.

### Background art

Medical implants are frequently implanted into vertebrate animals today to replace anatomy and/or restore functions of the body. One such type of implant is a dental implant.

Dental implants are utilized in dental restoration procedures in patients having lost one or more of their teeth. Dental implants come in many variants, including different numbers and types of component parts. However, dental implant structures generally comprise an anchoring portion, to be submerged and anchored in bone tissue, and an abutment post portion, which extends directly or indirectly from the anchoring portion for directly or indirectly supporting the dental prosthesis. The prosthesis, for example in the form of a crown or bridge, will hence be attached over, and supported by, the assembled dental implant structure.

The term "anchoring portion", as used herein, is the portion of the dental implant structure which is intended to be submerged in bone tissue. Typically, the anchoring portion will be provided with mechanical retention elements for primary stability within the bone and/or surface structuring for promoting osseointegration of the anchoring portion in bone tissue. An example of a mechanical retention element is a helical screw thread which may be selected with suitable forms and sizes. An example of suitable surface structuring is additive or non-additive roughening, e.g. blasted or chemically treated surfaces for promoting growth and/or adherence of bone cells to the surface. The anchoring portion is surgically implanted into the jawbone, where after bone tissue cells (osteoblasts) grow on and into the surface of the anchoring portion such that the implant is fixated in the bone with the bone in direct contact with the implant surface. This process is called osseointegration. By means of the osseointegration, a rigid installation of the implant is obtained.

The term "abutment post portion" as used herein, is the portion of the implant structure which, in use, protrudes into the oral cavity of the patient and provides support to a dental prosthesis, which can either take the form of an individual tooth prosthesis such as a crown, or a larger structure such as a bridge. The prosthesis comprises a cavity for accommodating the abutment post portion, such that, in use, the abutment post portion provides core support to the prosthesis. The prosthesis can either be attached directly to the abutment post portion, e.g. via bonding, screwing, direct veneering, or indirectly, for example via a sleeve or other covering which surrounds the abutment post portion. The abutment post portion is usually generally cylindrically, conically or frusto-conically shaped and has a length which enables this to extend into the prosthesis up to at least 50% of the prosthesis height.

The anchoring and abutment post portions can be integrally formed or may be formed on separate components for direct or indirect connection. When the portions are formed by separate components, each must comprise connection geometry which enables these to be fastened together, in some cases via an intermediate component.

In general, the component comprising the anchoring portion is often referred to in isolation as the implant. The implant can consist only of the anchoring portion, and hence be intended to be completely embedded in the bone, that is to say to the height of the alveolar crest. Alternatively, the implant can comprise other portions extending coronally of the anchoring portion. For example, the implant can comprise a coronal neck portion which is intended to protrude from the alveolar crest into the soft tissue, e.g. by a few millimetres. Also, as stated above, the implant can additionally comprise an integral abutment post portion intended to support a prosthesis, in which case, in use, this abutment post portion will extend through the soft tissue and into the oral cavity.

For the avoidance of doubt, the terms "apical" and "coronal" are used herein as is conventional in the art. That is, "apical" refers to a direction toward the jaw bone, or when referring to a portion of a component, to the portion that will be in the direction of the jaw bone when the component is correctly positioned in a patient's mouth. "Coronal" refers to a direction opposite the jawbone and towards the crowns of the teeth, or when referring to a portion of a component, to the portion that will be in the direction of the crowns of the teeth when the component is correctly positioned in a patient's mouth.

When the implant comprises an integral abutment post portion for supporting a prosthesis, this is sometimes referred to as a one-piece or one-part implant.

When the implant does not include an integral abutment post portion, this is provided as a separate component. The abutment component generally comprises the abutment post portion for directly or indirectly supporting a prosthesis, and a connection portion for connecting the abutment component to the implant component (or intermediate component). In most cases, one of the implant and abutment components will comprise a socket, or cavity, and the other a plug, or protrusion, which can be inserted into this socket. Often the connection geometry of each component will comprise complementary anti-rotation means having non-circular cross-sections, e.g. a polygonal contour or radially extending grooves or protrusions. The complementary anti-rotation means prevent relative rotation of the implant and abutment when fastened together, e.g. by bonding, force fit or a screw component.

A dental implant system comprising a separate abutment component to be connected to the implant is sometimes referred to as a multi-part implant. When the abutment connects directly to the anchoring component the system is usually referred to as a two-piece or two-part implant.

The abutment is mounted either directly or indirectly to the anchoring component after the latter has become incorporated (osseointegrated) into the bone or directly after the anchoring component has been inserted. It can also be attached to the anchoring component prior to insertion. Most usually the abutment is not mounted until after osseointegration. In such cases a component called a healing cap is often mounted to the implant during the osseointegration process to prevent incursion of soft tissue over the implant site.

In contrast to one piece implants, multi-part implants are more versatile, because the anchoring part and the abutment can be adapted to individual requirements. In particular the abutment shape and angulation, relative to the anchoring part, can be selected after implant insertion. This provides the surgeon with more flexibility and room for error in the placement of the implant. An additional advantage of multi-part implants is that the abutment can be made from a different material than the anchoring part.

Due to their versatility multi-part and particularly two-part dental implants are more commonly used than one-piece implants, and it is this form of implant system with which the present invention is concerned. For the remainder of this specification therefore, the term "implant" will be used to denote the anchoring component of a multi-part implant, namely, the element which in use is anchored within the bone but which does not directly provide core support to the final prosthesis, and the term "secondary component" will be used to denote a component which is, in use, directly or indirectly fastened to the implant. The secondary component can therefore be an abutment, which in use extends into the oral cavity and provides core support for a dental prosthesis, or may comprise an auxiliary component such as a healing cap. It can also be a superstructure component forming a single crown.

Once the anchoring portion of the implant structure has been introduced and osseointegrated into the jaw bone of the patient, it is necessary to prepare the prosthesis to be supported thereon.

In order to assist in creating the prosthesis, a model is usually made of the patient's mouth, which can be physical or virtual, and includes the position of the implant structure therein. The prosthesis can then be built with reference to this model. During the period between the making of the model and completion of the final prosthesis the patient is often provided with a temporary (also called a provisional) prosthesis. Such prostheses only remain in the patient's mouth for at most 6 months (180 days) and are not subjected to the full forces to which the final prosthesis will be exposed. They do however provide some chewing function and aesthetic effect to the patient while awaiting the final prosthesis. Although, in some cases, the temporary prosthesis could be fastened to the final abutment post portion, typically a temporary abutment is connected to the implant to support the temporary prosthesis. This is particularly the case when the temporary prosthesis is to be attached to the implant structure via bonding, as the removal of such prostheses is often not possible without damaging the underlying abutment structure.

Provisional components can be made from the same materials as the final implant components, e.g. metals, such as titanium and titanium alloys, ceramics and hard plastics such as PEEK (polyether ether ketone). However, due to the shorter time in the mouth and reduced strength requirement, other materials can also be used, such as PMMA (poly(methyl methacrylate)).

PMMA (poly(methyl methacrylate)) is a thermoplastic polymeric material formed by polymerising methyl methacrylate. PMMA has a good degree of compatibility with human tissue, and is often used as a temporary (provisional) prosthesis material. An advantage of PMMA is that it is easy to grind, thus allowing easy individualisation. In addition, it enables direct veneering, whereas other materials, such as PEEK, must first be primed with a binder material. Thus, PMMA is a convenient material for use in temporary dental secondary components, such as abutments.

PMMA is often modified to improve its properties, such as by modification with comonomers, additives, and fillers. For example, comonomers such as butyl acrylate can be added to improve the impact strength. PMMA may be dyed in order to colour it for different purposes and other filler materials used to increase cost effectiveness. In order to withstand the loads that will be applied to them, dental PMMA components are often strengthened via known cross-linking methods. In comparison to PEEK however, PMMA is relatively weak and therefore is used only in relation to temporary secondary components, such as temporary (provisional) abutments.

PMMA may be cross-linked with various components, for example a multifunctional methacrylate molecule (a molecule that carries two or more methacrylate groups), in order to link one polymer chain to another. This increases the strength of the polymer as well as reduces creep. The ability of crosslinking to fix the shape of PMMA is another reason for using this technique. The properties of the cross-linked product will vary depending on the degree of cross-linking, i.e. the ratio of crosslinker to monomer, as well as, to a lesser extent, on the cross-linking molecule used.

In addition to cross-linking, the strength of PMMA can be increased by using interpenetrating polymer networks (IPN). In such systems, two or more polymer networks (each created by cross-linking) are at least partially interlaced but not covalently bonded to each other. The two (or more) networks are thus mechanically connected but not chemically bonded. A cross-linked PMMA network can therefore be interlaced with one or more additional polymer networks to form IPN PMMA. The properties of the IPN PMMA will depend on the other polymer network(s) used. For example, creating an IPN PMMA with PMMA and poly(hydroxyethylmethacrylate) increases the hydrophilicity of the PMMA polymer.

While pure PMMA is a thermoplastic, that is, it can be melted and re-set, cross-linked, and hence IPN PMMA is thermoset. In other words, the polymer material is irreversibly cured and cannot be re-melted. Instead of melting, such plastics degrade or burn.

Dental implant components that have contact with body tissue, such as bone and soft tissue, need to be sterile when used because microbial contamination could result in disease transmission, microbial infection and/or inflammation. A number of different methods are available for sterilising components intended for implantation. The choice of method used depends on e.g. the material and/or design of the component to be sterilised.

Common methods for sterilisation include radiation sterilisation, chemical sterilisation, steam sterilisation (autoclaving), etc.

Radiation sterilisation is carried out by subjecting the object to be sterilised to radiation via e.g. electron beams, X-rays, gamma rays, or subatomic particles. The method is advantageous as it often does not affect the properties of the material sterilised. However, as exposure to radiation is hazardous, high requirements for environmental and working place safety are required.

Chemical sterilisation is often used for sterilising materials that are sensitive to heat. Chemical sterilisation for example involves subjecting objects to be sterilised to high concentrations of reactive gases e.g. alkylating agents, such as ethylene oxide or oxidizing agents, such as hydrogen peroxide and ozone. Liquid chemical sterilising agents include hydrogen peroxide, peracetic acid, and aldehydes. The use of chemical sterilisation however necessitates that the material to be sterilised is not affected or destroyed by the chemicals used. Further, the chemical agents used for sterilisation, due to their sterilising properties, are also dangerous to living beings and the environment. Thus, as with radiation sterilisation, high safety requirements are necessary.

Steam sterilisation, or autoclaving, involves subjecting objects to steam at a high temperature. Steam sterilisation involves the use of saturated steam under pressure and is an inexpensive and non-toxic method for sterilisation. Further, steam sterilisers (autoclaves) are available in different sizes for different purposes. Table-top steam sterilisers are commonly used e.g. by dentists in dental clinics. Four factors are of importance for a successful outcome of steam sterilisation: steam, pressure, temperature and time. Industry Standard steam sterilisation procedures require sterilisation at either 121°C or 132-134°C (see for example "Guideline for Disinfection and Sterilization in Healthcare Facilities, 2008", US Centers for Disease Control and Prevention, and "Bundesgesundheitsbl-Gesundheitsforsch-Gesundheitsschutz 11.2001" Robert Koch Institut, Germany). 121°C is the lowest acceptable sterilisation temperature as below this eradication of bacteria is not confirmed. On the other hand, 132-134°C is the upper limit at which standard autoclaves can operate.

The sterilisation time required varies depending on the product to be sterilised, but times of 20-30 min at 121°C and 4-6 min at 132-134°C for dental implant components are usual. Due to the higher temperature, the sterilisation time is lower at 132-134°C. Typically, at 121°C a sterilisation time of at least 15 min is necessary in order to achieve sterility, while at 132-134°C the sterilisation time may be as short as 3 min, or less. For a satisfactory result, it is important to ensure that all parts of the object to be sterilised reach the required temperature for the required time. Therefore, the sterilisation time necessary at a defined temperature will also depend on the material and objects sterilised. Chemical and biological indicators are available for monitoring the sterilisation process and to ensure that sterility is achieved. Properly executed steam sterilisation will inactivate all fungi, bacteria, viruses and bacterial spores. However, steam sterilisation may only be used for objects of which all parts can withstand the high temperature and pressure used for the procedure. For this reason, many objects are not suitable for steam sterilisation.

The glass transition, or softening, temperature of PMMA is approximately 105-120°C. This is the temperature at which the material changes from a hard, brittle state to a softer, more rubber-like, state. The variation in softening temperature is due to differences in the composition of PMMA polymers and, to a lesser degree, their method of production. However, despite these variables, the softening temperature is relatively constant. Cross-linking of PMMA and the use of IPNs does not significantly alter the softening temperature. This is because, regardless of the linking components or additional polymer networks added to the polymer, the inherent properties of the PMMA molecules themselves remain unchanged. Although thermoset forms of PMMA will not melt at the softening temperature, it is known that they will begin to degrade and undergo structural changes at this temperature. Due to its low softening temperature therefore, it is commonly accepted that PMMA is not suitable for the above discussed steam sterilisation methods as these have to be performed above the softening temperature.

Currently available PMMA dental components are therefore sold either for use with chemical cleaning procedures only, or after radiation sterilisation, in which case they are provided in a vacuum pack in order to preserve sterility. However, as explained above, there are drawbacks with such sterilisation methods and alternative methods for sterilisation of PMMA components would be advantageous.

In particular, most dental laboratories do not have the facilities to conduct radiation sterilisation. As PMMA components are often individualised before implantation, there is a need for the practitioner, such as the dentist or lab technician, to be able to modify a component, e.g. by individualising its shape, and thereafter sterilising or re-sterilising it before implantation. Providing pre-sterilised PMMA components is thus not sufficient in cases where the component has to be further modified before use, as such processes generally render the component unsterile, thereby necessitating re-sterilisation before implantation. One common method for sterilising PMMA components has therefore been to treat them with chemical agents/disinfectants in the practitioner's clinic before implantation as this is feasible in such environments. Radiation sterilisation is not an option for sterilisation in a practitioner's office and is thus only suitable for PMMA components which will be directly used, e.g. for implantation, without further modification after sterilisation.

The object of at least a preferred embodiment of the present invention is to overcome or at least mitigate some of the problems associated with the prior art.

### Summary of invention

According to one aspect, the present invention provides a method of sterilising a dental secondary component at least partially formed from thermoset poly(methyl methacrylate) (PMMA), comprising the step of subjecting the dental secondary component to steam sterilisation. An example of a dental secondary component is a temporary secondary component, such as an abutment, used in dental implantology procedures.

As PMMA has a glass transition, or softening, temperature of approximately 105-120°C at which the material changes from a hard, brittle state to a softer, more rubber-like, state, steam sterilisation has not been considered possible for articles comprising PMMA as this has to be carried out at temperatures above the glass transition temperature. This is particularly relevant for thermoset PMMA (such as cross-linked or IPN PMMA) as this material cannot be re-shaped but rather will degrade or burn when heated above the glass transition temperature.

Despite this prejudice in the art against using steam sterilisation for sterilising PMMA, it has now been surprisingly found that it is possible to sterilize PMMA-containing dental secondary components by steam sterilisation without causing a degradation of the PMMA material and without affecting its strength and biocompatibility. As demonstrated in the experimental section, steam sterilisation of thermoset PMMA-containing components did not affect the mechanical or biocompatibility properties of the PMMA. Thus, contrary to commonly held belief, the inclusion of thermoset PMMA within a dental secondary component does not prevent the use of steam sterilisation. Of course, should the component also contain a material having a softening or melting temperature less than PMMA it may be that steam sterilisation cannot be used. Thus, in the context of the present invention, the term "PMMA component" refers to a dental secondary component at least partially formed of thermoset PMMA, all other parts of the component being made of a material(s) having a softening temperature high enough to withstand steam sterilisation at the chosen sterilisation temperature. Generally, this means that the PMMA material has the lowest softening temperature of the materials found in the PMMA component.

This invention is therefore directed to a method of sterilising a thermoset PMMA (such as crosslinked or IPN PMMA) dental secondary component, the method comprising the step of subjecting the PMMA component to steam sterilisation, i.e. autoclaving. The invention therefore also is directed to the use of steam sterilisation for sterilising such a thermoset PMMA component.

Steam sterilisation is very advantageous as it opens up completely new possibilities of providing unsterile PMMA components to a practitioner, who can then sterilize the component before use or after further modification. Alternatively, sterile PMMA components may be provided which may be re-sterilised, for example after modification if such turns out to be necessary or if the component is handled in a manner making it non-sterile. Being able to provide the PMMA component in unsterile form is advantageous as it, e.g., makes the handling and transport of the component easier, which e.g. allows a reduction of costs. The use of steam sterilisation for PMMA components is also beneficial as it reduces the use of chemical agents which may cause problems for environmental and working place safety reasons. Also, steam sterilisation does not risk that remnants of chemicals are left on the components and transferred to a body when the PMMA component is implanted into the body, thereby reducing the risk for foreign body reactions such as allergy and inflammation.

By steam sterilisation, steam sterilising, autoclaving etc. is meant a sterilisation method involving subjecting objects to be sterilised to saturated steam under pressure thereby causing a high temperature to be effected. The terms steam sterilisation and autoclaving may be used interchangeably in the present document. Steam sterilisation (autoclaving) typically takes place in an autoclave. There are different kinds of steam sterilisers which may be used. The two basic types of steam sterilizers (autoclaves) are the gravity displacement autoclave and the high-speed prevacuum sterilizer. In a gravity displacement sterilizer, steam is let in at the top or the sides of the sterilising chamber and, because the steam is lighter than air, forces air out the bottom of the chamber through the drain vent. A high-speed prevacuum sterilizer, otherwise known as a dynamic-air-removal steriliser, is similar to the gravity displacement autoclave except that it also contains a vacuum pump that removes air from the sterilising chamber before steam is admitted. Steam sterilisation may involve a steam flush-pressure pulsing process, or fractionated vacuum procedure, in which air is rapidly removed by repeatedly alternating a steam flush and a pressure pulse above atmospheric pressure. Steam flash sterilisation cycles may also be used wherein the objects to be sterilised are subjected to higher temperatures for shorter time periods. All types of steam sterilisation can be used in the present invention. In general, gravity displacement systems are more common as these are less expensive.

By sterile, sterilisation and the like is in the present context meant obtaining an object which is free from microorganisms such as fungi, bacteria and viruses. A number of different tests are available for testing sterility, including mechanical tests for monitoring the sterilisation process, chemical indicators in the form of sensitive chemicals to assess physical conditions, such as temperature, during the sterilisation process and biological indicators that directly determine whether the most resistant microorganisms (e.g. *Geobacillus* or *Bacillus species*) are present rather than merely determine whether the physical and chemical conditions necessary for sterilisation are met. The person skilled in the art is well acquainted with the different options for monitoring sterilisation processes and how to determine if a satisfactory result is obtained. The probability of sterility for each item sterilised is commonly referred to as the sterility assurance level (SAL) of the product and is defined as the probability of a single viable microorganism occurring on a product after sterilisation. A generally accepted definition of sterility is an SAL level of 10⁻⁶, in other words, the probability of the sterilised item being non-sterile is one in a million. In contrast, disinfection is usually defined as an SAL level of 10⁻⁵.

As mentioned above, steam sterilisers (autoclaves) are available in different sizes and any type of autoclave may be used in the method of the invention. Table-top (portable) steam sterilisers are often used in outpatient, dental, and rural clinics. These sterilisers are designed for smaller instruments, such as syringes and needles and dental instruments. For sterilisation in a dental or medical clinic, thus typically a table-top autoclave will be used. Of course, also large scale autoclaves may be used, thereby enabling the sterilisation of a large number of PMMA components simultaneously, which may then be sold in sterile packing for direct use or optionally for modification and/or resterilisation.

In order to affect a sterilisation when using steam sterilisation, a temperature of at least 121°C is considered necessary. The time of sterilisation will depend on the type of product to be sterilised. Typically, use of a temperature of 121°C to sterilise dental secondary components requires that this temperature is held for at least 15 minutes, typically about 20-25 minutes, in order for sterility to be obtained. Alternatively, a higher sterilisation temperature may be used, thus allowing the holding time at the sterilisation temperature to be reduced. At 132-134°C the sterilisation time may be as low as 3-5 minutes.

The present document presents tests demonstrating that thermoset PMMA components can be steam sterilised at a temperature of 134°C up to a time of 30 minutes without any reduction in their ability for use as a temporary dental secondary components. In other words, the required fatigue and biocompatibility tests are passed after a steam sterilisation procedure at 134°C for 30 minutes.

Therefore, preferably said steam sterilisation is carried out at a temperature between 121 and about 134°C for a time of up to 30 minutes.

In a preferred embodiment, a temperature of 121°C is used for a time of at least 15 min, preferably between 20 and 30 minutes, more preferably 20-25 min, such as 20, 22, 24, 26, 28 or 30 min. In an alternative preferred embodiment, steam sterilisation may be carried out at a temperature of between 132-134°C for at least 3 min, preferably between 3 and 6 minutes, such as 3, 4, 5 or 6 min. Although tests have shown that sterilisation at 134°C for 30 minutes produces no negative effect on the thermoset PMMA components, it is preferred that sterilisation at 134°C lasts no longer than 15 minutes. This is to ensure that the component can be re-sterilised if necessary, either after grinding or following accidental contamination, without the total sterilisation time exceeding 30 minutes.

By "thermoset PMMA" is meant a form of polymeric material "poly(methyl methacrylate)", [C₅H₈O₂]ₙ, which is irreversibly cured, i.e. it cannot be re-melted. Thermoset PMMA includes cross-linked PMMA, e.g. by a multifunctional methacrylate molecule, and PMMA comprising interpenetrating polymer networks. As mentioned above, in PMMA comprising such interpenetrating polymer networks, two or more polymer networks (each created by cross-linking, and of which at least one is based on PMMA) are at least partially interlaced but not covalently bonded to each other. In this document, IPN PMMA will be used for denoting this kind of PMMA.

The thermoset PMMA component preferably comprises a cross-linked PMMA. In some embodiments the PMMA may further be part of an interpenetrating polymer network (IPN PMMA). The PMMA is preferably cross-linked by a multifunctional methacrylate molecule, such as a dimethacrylate molecule, e.g. ethyleneglycoldimethacrylate. However, it is also possible to use cross-linking components having more than two methcrylate groups, in which case a lesser amount of cross-linker will be necessary to achieve the same degree of cross-linking, as each molecule can bond three or more polymer chains, rather than only two. In addition, gamma radiation can also be used to crosslink PMMA.

Preferably, the PMMA material of the component comprises at least 50 wt% methylmethacrylate and up to 50 wt% cross-linker, preferably a multimethacrylate cross-linker. More preferably, the PMMA material comprises at least 75% methylmethacrylate and up to 25 wt% crosslinker, preferably a multimethacrylate cross-linker. The PMMA material may additionally comprise additives such as comonomers, dyes and other fillers. In one embodiment, the thermoset PMMA used in the present invention comprises at least 95 wt% cross-linked PMMA. Preferably the thermoset PMMA used in the present invention has a filler content of less than 20 wt%, preferably less than 15 wt%, most preferably between 10-15 wt%. Example of fillers which might be used include dyes, such as titanium oxide, aluminium oxide, titanium-dioxide (rutile) and nickel-rutile.

As mentioned above, the cross-linking and, where included, IPN used does not generally alter the softening temperature of a PMMA component, and is therefore generally not important for successfully using the methods of steam sterilisation disclosed herein.

Preferred thermoset PMMA materials for use with steam sterilisation include VITA® CAD-Temp®, produced by VITA Zahnfabrik, and polycon® ae, available from Institut Straumann AG. The former material is an example of cross-linked PMMA and the latter of an IPN PMMA.

Preferably, the thermoset PMMA used in the present invention has a softening temperature of between 105-120°C. More preferably, the thermoset PMMA has a softening temperature of between 110 and 120°C, such as 110, 112, 114, 116, 118 or 120 °C.

The thermoset PMMA components used in the present invention are dental secondary components for direct or indirect connection to a dental implant. Such components include temporary abutments, healing caps and temporary superstructures, such as crowns. The secondary components for use in the present invention are preferably components suitable for use in single tooth restorations, i.e. those small enough to be fully supported on a single dental implant. This is because, although the steam sterilisation will not affect the strength or biocompatibility of the PMMA components, slight deformations in shape and dimensions may occur. The size of single tooth secondary components is such that any deformation is so slight that it does not affect the functionality or appearance of the component.

A secondary component suitable for use in single tooth restorations is any component capable of being fully supported by a single implant structure, i.e. without additional support provided by another object, such as a second implant, tooth or tooth stump. The secondary component can be a component used as part of a single tooth restoration, such as an abutment or crown, or an auxiliary part used during the preparation of the restoration, such as a healing cap. In general, the maximum length of the PMMA part of a secondary component suitable for use in single tooth restorations is 20 mm and the maximum diameter is 15 mm.

When dental secondary components have a load bearing function, such as when the component is a temporary abutment, it is required that the PMMA has a flexural strength of at least 80 MPa.

Therefore, preferably the thermoset PMMA used in the present invention have a flexural strength of at least 80 MPa, preferably between 80 and 150 MPa, most preferably 80-100 MPa, such as about 80, 85, 90, 95 or 100 MPa. A material's "flexural strength" is the material's ability to resist deformation under load.

Preferably, the thermoset PMMA used in the present invention has a flexural modulus of at least 2500 MPa, preferably between 2500-3000 MPa. In short "flexural modulus" refers to the ratio of stress to strain in flexural deformation, or the tendency for a material to bend.

Preferably the thermoset PMMA used in the present invention has an elastic modulus of at least 2500 MPa, preferably between 2500-3000 MPa. The "elastic modulus" is a measure of an elastic material's stiffness.

Tests for determining the flexural strength, flexural modulus and elastic modulus are well-known to a person skilled in the art.

Although the present invention enables the use of steam sterilisation as a means for sterilising PMMA components, it may in some instances be advantageous to complement this steam sterilisation with cleaning using chemical cleaning agents, typically before carrying out the steam sterilisation. Typical cleaning agents include agents which destroy and/or kill microorganisms and include, but are not limited to, alcohols (e.g. ethanol or propanol), aldehydes (e.g. glutaraldehyde) and/or oxidising agents (e.g. hydrogen peroxide, iodine or ozone). A cleaning agent may be beneficial to use to destroy or kill microorganisms or remove dust or dirt or tissue remnants from a PMMA component. Importantly, the cleaning agents used should not leave harmful residues on the PMMA component. Preferably, therefore the method further comprises the step of cleaning the PMMA component with one or more cleaning agents, wherein the cleaning step is carried out before steam sterilisation. A preferred cleaning agent is potassium hydroxide. In a preferred embodiment the cleaning agent contains a surfactant. Neodisher® Mediclean Dental, produced by Dr Weigert, is a suitable cleaning agent. An alternative preferred cleaning agent is orthophthalaldehyde, such as CIDEX® OPA solution, produced by Johnson and Johnson.

Additionally, or alternatively, the method of the present invention can comprise a disinfecting step, wherein the disinfecting step is carried out before steam sterilisation and, if a chemical cleaning step is used, after said cleaning step. Preferably, the disinfecting step comprises (e.g. dry) heating the component to between 80-100°C, preferably between 90-95°C.

Additionally, or alternatively, the method can comprise the step of modifying the external shape of the PMMA component such as via grinding. Preferably, said modifying step occurs prior to said sterilisation step, however, in other embodiments the modifying step takes place after a first sterilisation step (preferably carried out by steam sterilisation although another sterilisation method may also be used for the first sterilisation step), whereupon a second sterilisation step follows the modifying step, the second sterilisation step comprising steam sterilisation.

As discussed above, the PMMA component is a secondary component of a dental implant system, for direct or indirect connection to a, preferably single, dental implant implanted in a human or animal body. The PMMA component can be, for example, a gingiva former or an abutment for a dental implant, such as a temporary abutment. The component can consist solely of thermoset PMMA, however the component may also comprise other parts, not made of or comprising PMMA, which can withstand steam sterilisation. In a preferred embodiment, the dental secondary component is designed for direct connection to a dental implant, in other words, it directly contacts the implant. In such preferred embodiments the component preferably comprises an inlay formed of a harder material than the thermoset PMMA. The inlay preferably comprises connection geometry at its apical end for direct connection to the dental implant and can further comprise engagement geometry at its coronal end for connection to the thermoset PMMA material. The connection geometry can be located outside the PMMA, for example, when the apical end of the inlay is arranged for insertion into a bore of an implant. Alternatively, the connection geometry might be located within the PMMA material, for example, when the apical end of the inlay is arranged to be attached to a protruding boss of an implant. Providing a connection between the implant and an inlay creates a more secure connection and reduces the likelihood of damage to the secondary component which could occur if the softer PMMA was in load bearing contact with the harder material of the implant. In addition, providing implant connection geometry on the inlay prevents any dimensional changes which occur to the PMMA during steam sterilisation from affecting the fit between the implant and component. The implant connection geometry preferably comprises anti-rotation means, e.g. a section having a non-circular cross section, for preventing relative rotation between the implant and component.

The inlay is preferably formed of metal, such as titanium or titanium alloy, e.g. TAN (Ti-6Al-7Nb). In other embodiments, the inlay can be formed of ceramic. The PMMA can be directly polymerised, bonded, screwed, or press fit to the inlay such that at least part of the inlay is located within the PMMA material.

According to another aspect the present invention provides a kit comprising one or more dental secondary component(s) at least partially formed from thermoset PMMA as disclosed herein and one or more tray(s) for housing said dental secondary component(s) during the steam sterilisation process as disclosed herein. The kit thus comprises or consists of at least one dental secondary component(s) at least partially formed from thermoset PMMA and at least one tray for steam sterilisation. Optionally, such a kit may also comprise other parts such as instructions for sterilisation and/or cleaning agents as disclosed elsewhere herein. Also, the kit may include a substance for promoting healing and/or regeneration of soft and/or hard tissue, such as enamel matrix proteins, e.g. in the form of Straumann® Emdogain. The material for the tray is not limited as long the material withstands the conditions of steam sterilisation. A suitable material for the tray is metal, such as steel, or hard polymer, such as PEEK.

The tray may have a number of different designs but importantly, the design allows the thermoset PMMA component to be handled after sterilisation without risk for contaminating the thermoset PMMA component. The tray may thus e.g. have a bowl shape, or the shape of a closed container. Each tray could be designed to encompass one, two or more PMMA components.

According to a further aspect, the present invention provides a kit comprising one or more dental secondary component(s) at least partially formed from thermoset PMMA as disclosed herein and an apparatus for steam sterilisation as disclosed herein, such as a table-top autoclave. Optionally, such a kit may also comprise other parts such as instructions for sterilisation, cleaning agents and/or a substance for promoting healing and/or regeneration of soft and/or hard tissue as disclosed elsewhere herein.

In accordance with yet another aspect, the present invention provides a sealed, non-sterile container, such as the tray for housing the PMMA component(s) during the steam sterilisation described above, comprising an unsterile dental secondary component at least partially formed from thermoset PMMA for sterilisation by steam sterilisation. The sale of unsterile PMMA components reduces the cost and complexity of handling and packaging these components. As the parts can be steam sterilised in a dental lab using a standard table top autoclave it is possible for the end user, namely the dentist or dental surgeon, to quickly and simply sterilise the products prior to use, and/or after any desired modification to the product shape. Previously PMMA components have been sold in vacuum packs after sterilisation via radiation.

In accordance with a further aspect, the present invention provides a kit comprising one or more dental secondary component(s) at least partially formed from thermoset PMMA as disclosed herein and instructions for sterilisation of said component by steam sterilisation at a temperature of between 121-134°C for up to 30 minutes.

The present invention is also directed to a sterile dental secondary component(s) at least partially formed from thermoset PMMA (as disclosed herein) which has been sterilised by steam sterilisation process as disclosed herein.

The present invention is therefore also directed to a sterile dental secondary component(s) at least partially formed from thermoset PMMA (as disclosed herein) obtained by the process of subjecting an unsterile dental secondary component(s) at least partially formed from thermoset PMMA to a steam sterilisation method as disclosed herein. During steam sterilisation, water is absorbed by the PMMA component, thus slightly changing the water content and shape of the PMMA component. The water absorption according to ISO 10477 of a PMMA dental component should be less than 26.5 µg/mm³. For an abutment comprising a PMMA part of 11 mm in height this allows for an increase in height of the PMMA part by approximately 0.1 mm, i.e. about 0.9%. The height of the PMMA steam sterilised as disclosed herein therefore should generally not change its length by more than about 1%. Measurements before and after steam sterilisation as disclosed herein of PMMA components confirm that these criteria are fulfilled and that steam sterilisation therefore successfully can be used for sterilising single tooth dental PMMA components.

### Brief description of drawings

Preferred embodiments shall now be described, by way of example only, with reference to the accompanying drawings, in which:
FIG 1 shows an exemplary temporary dental secondary component for use in the present invention;
FIG 2 shows a further exemplary temporary dental secondary component for use in the present invention;
FIG 3a shows a flow chart outlining a method in accordance with a preferred embodiment of the present invention;
FIG 3b shows details of step 2 of the method outlined in Fig. 3a; and
FIG 4 shows a flow chart outlining another method in accordance with a preferred embodiment of the present invention.

### Detailed description of the invention

Components made of, or comprising, thermoplastic and thermoset PMMA have previously been used for dental prosthesis components, in particular crowns, bridges, temporary abutments and gingiva formers for direct or indirect connection to a dental implant.

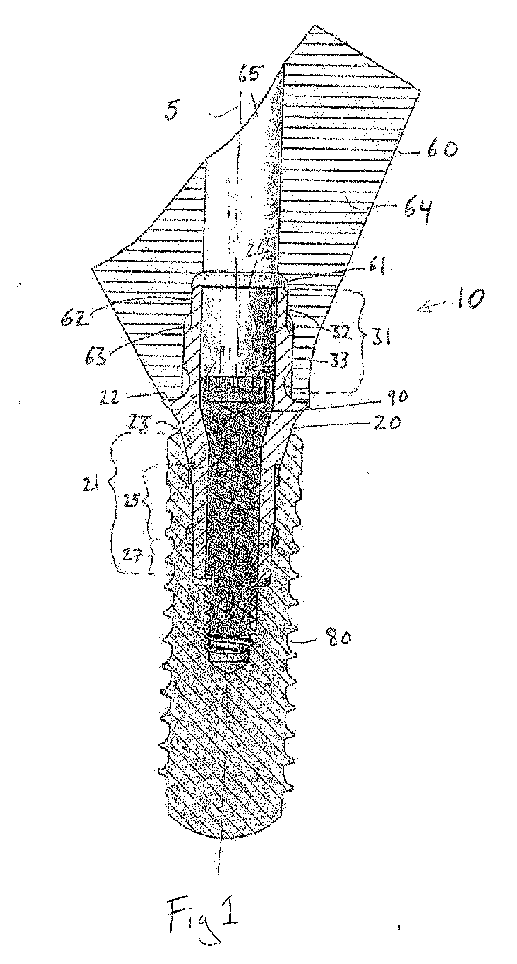

FIG 1 shows an example of such a component. Temporary abutment 10 is intended to be connected to an implanted implant after osseointegration but prior to the completion of the final prosthesis. It comprises an inlay 20 and overlay 60. The inlay comprises apical portion 21 having implant connection geometry. The apical portion 21 is shaped for insertion into an internal bore of a dental implant 80. In alternative embodiments, the apical portion can instead be shaped to accommodate a coronally protruding boss of the implant.

Apical portion 21 comprises a conical portion 23 tapering in the apical direction and, apical of conical portion 23, anti-rotation means 25. In the present embodiment anti-rotation means 25 comprises a series of grooves and flats, although this could alternatively be any shape having a non-circular cross-section, e.g. a hexagon, octagon etc. The grooves and flats are positioned and dimensioned to co-operate with a number of protrusions within the bore of the implant, such that the inlay 20, and hence the temporary abutment 10 as a whole, is held in a rotationally locked position relative to the implant 80. Apical portion 21 further comprises, apical of the anti-rotation means 25, circular cylindrical guidance portion 27 which helps to centre the inlay 20 during insertion into the implant.

Coronal of the apical portion 21, the inlay 20 comprises a coronally facing platform 22 which acts as an abutment surface and axial stop for the overlay 60.

Post portion 31 extends coronally from platform 22 along longitudinal axis 5. This portion can comprise grooves and/or protrusions 33 or have a non-circular cross-section, e.g. oval or polygonal, such that the overlay can be connected to this in a non-rotational manner.

The inlay 20 is formed of a hard material, such as metal or ceramic, to complement the hardness of the implant 80 to which it will be connected.

Overlay 60 comprises accommodation cavity 61 at its apical end. Cavity 61 is formed of circular cylindrical cavity wall 62 from which radially extending grooves 63 extend into the overlay. Circular cylindrical wall 62 is dimensioned to complement circular cylindrical external surface 32 of the inlay such that the inlay fits snugly within the accommodation cavity 61. Grooves 63 are arranged evenly circumferentially spaced about the longitudinal axis 5 of the cavity 61 and are shaped to accommodate protrusions 33 of the inlay. In this embodiment the overlay 60 is bonded to the inlay 20, although other connection methods are possible, e.g. press-fit, screwing etc.

Abutment post portion 64 extends at an angle to longitudinal axis 5, thus forming an angled abutment. It is the abutment post portion 64 which will provide core support to the temporary prosthesis in use. Through hole 65 enables a basal screw 90 to be inserted through overlay 60, so that the abutment 10 can be temporarily connected to the implant 80.

Overlay 60 is formed of thermoset PMMA, which enables the outer surface of the overlay to be individualised through grinding. In addition, PMMA enables direct veneering, such that the temporary prosthesis can be veneered directly onto the abutment 10.

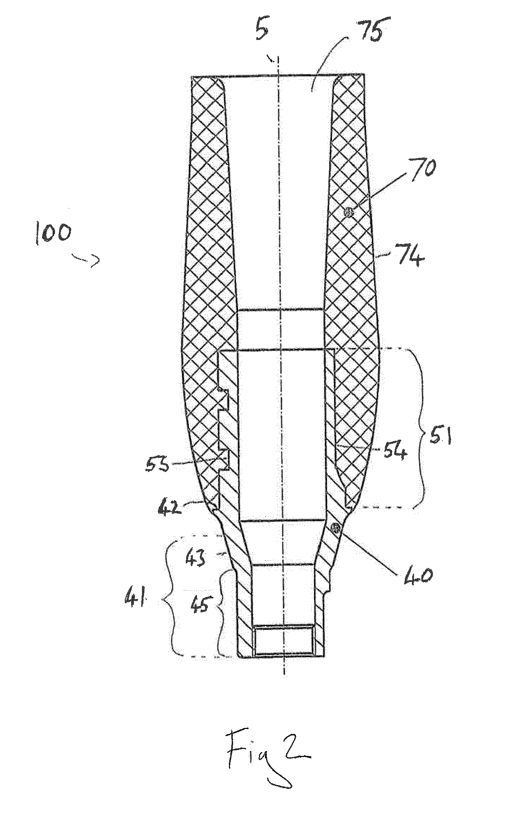

Fig. 2 shows an alternative temporary abutment 100. Once again, the abutment 100 comprises a metal inlay 40 having an apical portion 41 comprising implant connection geometry. In this embodiment the apical portion 41 comprises only a conical portion 43 and, apical of conical portion 43, anti-rotation means 45. Coronal of the apical portion 41, the inlay 40 comprises a coronally facing platform 42 which acts as an abutment surface and axial stop for the overlay 70.

Post portion 51 extends coronally from platform 42 along longitudinal axis 5. This portion is generally circular cylindrical, however a number of axially spaced grooves 53 and a longitudinally extending bevel 54 are machined into this surface.

Overlay 70 is formed of thermoset PMMA and is directly polymerised over the post portion 51 such that this is permanently joined to the inlay 40 in a non-rotational manner.

Abutment post portion 74 extends coaxial to longitudinal axis 5, thus forming a straight abutment. It is the abutment post portion 74 which will provide core support to the temporary prosthesis in use. Through hole 75 enables a basal screw to be inserted through overlay 70, so that the abutment 100 can be temporarily connected to the implant. Components that are intended for implantation in a tissue (as opposed to e.g. a crown which is placed above tissue) need to be sterile when inserted into the body in order to reduce the risk of microbial infection and inflammation. As PMMA components are often are individualized before implantation, there is a need for the practitioner, such as the dentist or doctor, to be able to modify a component, e.g. by individualizing its shape, and thereafter sterilising or re-sterilising it before implantation. Providing pre-sterilised PMMA components is thus not sufficient in cases where the component has to be further modified before use, as such processes generally render the component unsterile, thereby necessitating re-sterilisation before implantation.

One common method for sterilising PMMA components has therefore been to treat them with chemical agents/disinfectants in the practitioner's clinic before implantation as this is feasible in such environments. Radiation sterilisation is not usually an option for sterilisation in a practitioner's office and is thus only suitable for PMMA components which will be directly used, e.g. for implantation, without further modification after sterilisation.

It has now surprisingly been found however that, in spite of the low softening temperatures of thermoset PMMA, it is possible to steam sterilise dental secondary components comprising thermoset PMMA at temperatures of between 121-134°C for times of up to 30 minutes. Contrary to commonly held belief, such sterilisation processes do not have a negative impact on the strength or biocompatibility of these components, and hence do not impact on their ability to be used as temporary dental secondary components.

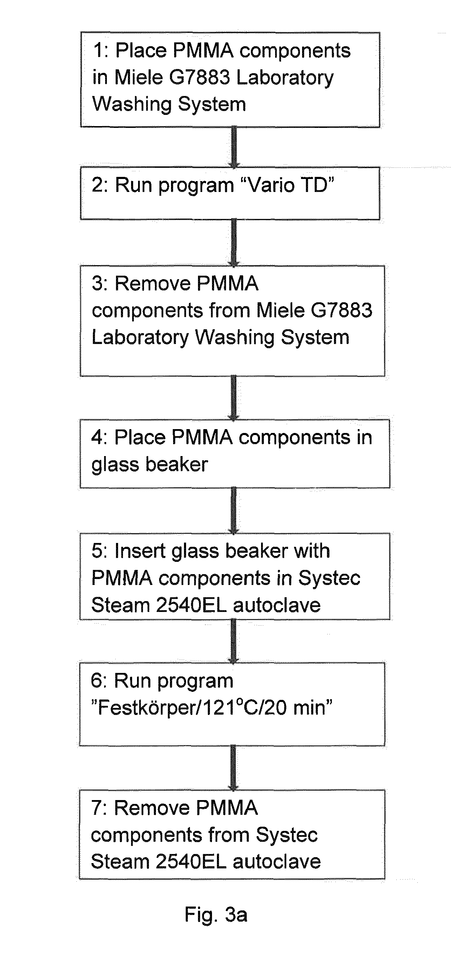

FIG 3a shows a flow chart of an exemplary sterilisation process in accordance with the present invention. The method comprises a pre-cleaning and disinfecting of the PMMA components before these are subjected to steam sterilisation. The cleaning agent used in this exemplary sterilisation method is neodisher® MediClean (Dr Weigert). In step 1 in the method outlined in Fig. 3a, PMMA secondary components, such as the ones shown in Figs. 1 and 2, are placed in a Miele G7883 Laboratory Washing System, which is tested and verified according to ISO 15883-1. Program "Vario TD" is then run (step 2). The PMMA components are thereafter removed from the Miele G7883 Laboratory Washing System (step 3) and placed in a clean glass beaker (step 4). The PMMA components in the glass beaker are thereafter inserted in a Systec Steam 2540EL autoclave (step 5) and the program "Festkörper/121°C/20 min" run (step 6). When step 6 is finalised, the PMMA components are removed from the autoclave (step 7) and sterile and ready to use.

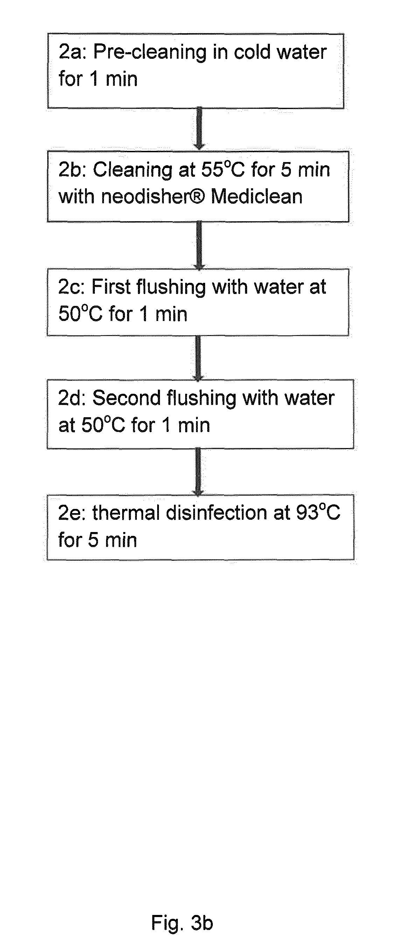

In Fig. 3b, the steps of the program "Vario TD", referred to in step 2 of the method outlined in Fig. 3a, are further illustrated. The program "Vario TD" starts with a pre-cleaning in cold water for 1 min (step 2a), followed by a cleaning at 55°C for 5 min with 0.2% neodisher® MediClean (step 2b), a first flushing with warm water at 50°C for 1 min (step 2c), a second flushing with warm water at 50°C for 1 min (step 2d), and as a last step a thermal disinfection at 93°C for 5 min (step 2e).

It is to be understood that the flow-charts of Figs. 3a and b are illustrative only and that methods and/or apparatuses providing the same or similar cleaning/sterilisation steps may be used instead. In particular, a steam sterilisation temperature of 134°C can be used for a time period of up to 30 min.

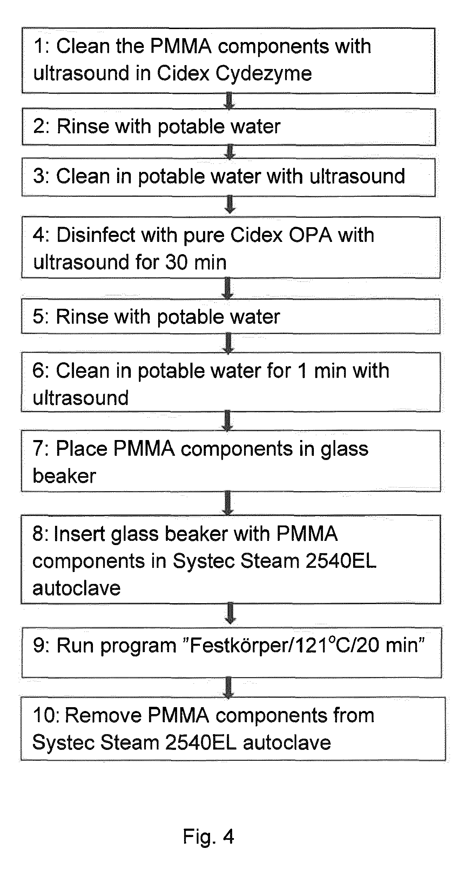

Fig. 4 is a flow-chart of a manual cleaning procedure wherein Cidex Cydezym is used as a cleaning agent and Cidex OPA is used as a disinfecting agent (both from Johnson & Johnson). Step 1 of this exemplary manual cleaning procedure is a cleansing step of the PMMA components in ultrasound with 1.6% Cidex Cydezyme (Johnson & Johnson) for 15 minutes. The PMMA components are thereafter rinsed with potable water (step 2) before a further cleaning step in potable water for 1 min with ultrasound (step 3). The PMMA components are then subjected to disinfecting in pure Cidex OPA (Johnson & Johnson) by ultrasound for 30 min (step 4) before being rinsed with potable water (step 5) and then cleaned in potable water for 1 min with ultrasound (step 6). The PMMA components are placed in a clean glass beaker (step 7) which is inserted in a System Steam 2540EL autoclave (step 8). The PMMA components are steam sterilised using the program "Festkörper/121°C/20 min (step 9). The PMMA components are thereafter sterile and ready to use after removal from the System Steam 2540EL autoclave (step 10). It is to be understood that the flow-chart of Fig. 4 is only exemplary and other methods and apparatuses providing the same or similar cleaning/sterilisation steps may be used instead. In particular, a steam sterilisation temperature of 134°C can be used for a time period of up to 30 min.

When the PMMA components are to be sterilised at a higher temperature and/or a different time, the same process as described above in relation to Figs. 3a, 3b and 4 can be followed, the only alteration being at step 9 of Fig. 4 or step 6 of Fig. 3a, in which the autoclave would instead be run at 134°C for a pre-selected time of up to 30 minutes.

The invention will be further described in the following examples, which do not limit the scope of the invention described in the claims.

### Experimental section

The dynamic tests performed in the below experiments comprise setting up a test model of a dental implant structure. In the present case, provisional abutments were tested and hence the dental implant set up comprised an implant and a provisional abutment, connected together using a basal screw. The implant model was tested according to ISO 14801:2007. During this test, the model is subjected to a predetermined "acceptable load". The acceptable load is not defined in the ISO Standard and instead is set according to the size and shape of the implant parts to be tested; for example, abutments intended for use in the molar region are typically tested at a higher acceptable load level than abutments for use in the anterior region. The acceptable load should mimic the forces that will be placed on the component during chewing. The test is performed according to the above mentioned ISO 14801:2007 and FDA Guidance (Doc.Nr 1389). In a deviation from the test method described in these documents however, the number of loading cycles undergone by the samples was 200,000 as opposed to the 2 x 10⁶ specified in the ISO standard. This change was made as the Standard applies to components intended for permanent placement in the mouth, as opposed to temporary components whose maximum use time is 6 months. Consequently, the load cycles were reduced.

A variety of PMMA secondary components, formed using different PMMA material and having different dimensions, were steam sterilised and then tested according to the above method. These components were compared to identical non-steam sterilised components which had undergone the identical test. All the steam sterilised components passed this test, demonstrating that the steam sterilisation did not compromise the mechanical strength of the PMMA in a way making it not practically useful in dental secondary components. In addition, steam sterilised PMMA components were also tested for biocompatibility. Once again, all the components passed this test.

### Example 1: Dynamic Test of Provisional Abutments comprising Polycon® ae PMMA sterilised at 121°C

### Materials and methods

In this example, a provisional abutment was tested which comprised a TAN (Ti-6Al-7Nb) inlay and bonded PMMA overlay, having a similar appearance to that of component 10 shown in Fig. 1. The PMMA material used was Polycon® ae (available from Institut Straumann AG). Polycon® ae is a PMMA material made of poly(methyl methacrylate) (>99.5%), titanium dioxide (<0.15%), antiom-/nickel-titaniumdioxide-rutile (<0.03%), yellow iron oxide (<0.03%), brown iron oxide (<0.006%), black iron oxide (<0.002%), red iron oxide (<0.0004%), and benzoyl peroxide (<0.45%). The poly(methyl methacrylate) is made of methyl methacrylate (>76%) crosslinked with ethylene glycoldimethacrylate (<20%) and further comprising allylmethacrylate (<2%) and organic luminescent pigments (terephtal acid ester) (<1%) as additives.

**Table 1: Sample Information**

| *part(s)* | *specification* | *number* |
|---|---|---|
| implant | Straumann BL, ø4.1 mm RC, SLActive 12 mm | 6 |
| prosthetics | RC TAN inlay (ref. no. 20 of Fig 1) | 6 |
| | Polycon® AE Provisional Coping (ref. No. 60 of Fig 1) | 6 |
| | RC Basal screw (ref. no 90 of Fig 1) | 6 |
| auxiliaries | loading hemisphere, radius 6 mm | - |

The PMMA coping was bonded to the TAN inlay using self-adhesive dental cement.

### Cleaning and Disinfection

The six components tested were first cleaned and disinfected. The test material was placed in a cleaning tray and positioned in a washer-disinfector (disinfector G 7836 CD, Miele, Gütersloh). The automated cleaning was carried out with the use of neodisher® MediClean forte (Dr Weigert) as cleaning solution. The thermal disinfection was carried out at 93°C for 5 min. The used program is based on vario TD (see elsewhere in this document). After cleaning/disinfection the test material was removed from the washer-disinfector under clean conditions. Cleaning and disinfection was performed once.

### Sterilisation

Steam sterilisation was carried out at 121°C with a holding time of 20 min using gravity-displacement as the steam sterilisation cycle.
- **The test equipment/Test device:**:
- Hydropulser Walter & Bai: QS-51806 (138)
QS-52225 (711)
QS-54861 (1012)
- **Test method:**: System test according to ISO 14801:2007 and FDA Guidance (Doc.Nr 1389.pdf)
- **Test specification:**: The load F was applied via a load rod at a 40° angle to the implant axis.
- **Applied load:**: The mounted components are cyclically tested on compression. Run outs: 200,000 cycles at 2 Hz.
- **Load level:**: 3 samples at predetermined Acceptable Load and 3 samples at predetermined Breakage Load
- **Test ambience:**: ambient air, room temperature

**Table 2: Results**

| **sample ID** | **load** | **No. of cycles** | **Failure mode / comment** |
|---|---|---|---|
| B724_C1 | Acceptable | 200'000 | *No damage* |
| B724_C2 | Acceptable | 200'000 | *No damage* |
| B724_C3 | Acceptable | 200'000 | *No damage* |
| B724_C4 | Breakage | 127'628 | *Abutment broke at screw head level* |
| B724_C5 | Breakage | 103'275 | *Abutment broke at screw head level* |
| B724_C5 | Breakage | 192'879 | *Abutment broke at screw head level* |

All devices passed the test at the acceptable load level. At the breakage load level all abutments broke at screw head level.

### Conclusion:

The test showed that steam sterilised provisional abutments comprising PMMA in the form of Polycon® ae are strong enough to pass the 200,000 cycles test at a predetermined acceptable load level. When a predetermined breakage load was applied, the abutments broke at a consistent location. The steam sterilized components thus performed in a similar manner as identical provisional abutments comprising Polycon® ae which had not been subjected to steam sterilisation, demonstrating that the properties of the PMMA containing abutments were not negatively affected by the steam sterilisation.

### Example 2: Dynamic Test of Provisional Abutments comprising VITA® CAD-Temp ® PMMA sterilised at 121°C and 134°C

### Materials and methods

In this example a provisional abutment was tested which comprised a TAN inlay and directly polymerised PMMA overlay, having a similar appearance to that of component 100 shown in Fig. 2. The PMMA material used was VITA® CADTemp® (available from VITA Zahnfabrik). VITA® CADTemp® is a microfiller reinforced polyacrylic material made of a high-molecular and cross-linked acrylate polymer with inorganic microparticle fillers polymerized into the network.

**Table 3: Sample Information:**

| *part(s)* | *specification* | *number* |
|---|---|---|
| implant | Straumann BL NC Ø3.3, 12mm Ti Gr.4, SLA coated, threaded | 9 |
| prosthetics | NC TAN inlay (ref. no. 40 of Fig 2) | 9 |
| | VITA CAD-temp Provisional Coping (ref. No. 70 of Fig 2) | |
| | NC Basal screw | 9 |
| auxiliaries | Loading hemisphere, radius 6 mm | - |

### Cleaning and Disinfection

Cleaning and disinfection was performed once as disclosed in Example 1.

### Sterilisation

Steam sterilisation of 6 samples [Lot A] was carried out at 121 °C with a holding time of 20 min using gravity-displacement as the steam sterilisation cycle.

Steam sterilisation of 3 samples [Lot B] was carried out at 134°C with a holding time of 30 min using gravity-displacement as the steam sterilisation cycle.
- **Test equipment/Test device:**:
- Hydropulser Walter & Bai: QS-51431 (389)
- **Test method:**: System test according to ISO 14801:2007 and FDA Guidance (Doc.No 1389.pdf)
- **Test specification:**: The load F was applied via a load rod at a 30° angle to the implant axis.

**Applied load:** The mounted components are cyclically tested on compression. Run out 200,000 cycles at 2 Hz.
- **Load level:**: **Lot A:** 3 at Acceptable Load and 3 at Breakage Load
**Lot B:** 3 at Acceptable Load
- **Test ambience:**: 0.9% NaCl, 37°C

**Table 4: Result**

| **Lot A** | | | |
|---|---|---|---|
| **sample ID** | **load** | **No. of cycles** | **Failure mode / comment** |
| B765_A10 | Acceptable | 200'000 | *No damage* |
| B765_A11 | Acceptable | 200'000 | *No damage* |
| B765_A12 | Acceptable | 200'000 | *No damage* |
| B765_A7 | Breakage | 139,856 | *Abutment broke at screw head level* |
| B765_A8 | Breakage | 18682 | *Abutment broke at screw head level* |
| B765_A9 | Breakage | 189,401 | *Abutment broke at screw head level* |

| **Lot B** | | | |
|---|---|---|---|
| **sample ID** | **load [N]** | **No. of cycles** | **Failure mode / comment** |
| B790_A1 | Acceptable | 200'000 | *n*/*a* |
| B790_A2 | Acceptable | 200'000 | *n*/*a* |
| B790_A3 | Acceptable | 200'000 | *n*/*a* |

### Conclusion:

The test showed that the provisional abutments comprising PMMA in the form of VITA® CAD-Temp®, sterilised at 121°C for 20 minutes are strong enough to pass the 200'000 cycles test at a predetermined acceptable load level. When a predetermined breakage load was applied, the abutments broke at a consistent location. The steam sterilized components thus performed in a similar manner as identical provisional abutments comprising VITA® CAD-Temp® which had not been subjected to steam sterilisation, demonstrating that the properties of the PMMA containing abutments were not negatively affected by the steam sterilisation.

Sterilisation of the same components at 134°C showed no influence on the mechanical material strength of the PMMA abutment, because the PMMA abutments sterilised at 134°C also passed the 200'000 cycle fatigue test at the acceptable load level.

### Example 3: BiocompatibilityTest of polycon® ae sterilised at 121°C

Provisional abutments of the type used in Example 1, containing the PMMA material polycon® ae, available from Institut Straumann AG, were subjected to biocompatibility evaluations and was found to be safe for its intended use.

In the present example, the biocompatibility of polycon® ae after cleaning, disinfection and steam sterilisation is tested. The biocompatibility testing was performed on samples subjected to a single cycle of cleaning, disinfecting and sterilisation (1x reprocessed samples) as described below.

### Cleaning and Disinfection

Test material was placed in a cleaning tray and positioned in a washer-disinfector (disinfector G 7836 CD, Miele, Gütersloh). The automated cleaning was carried out with neodisher® MediClean forte (Dr Weigert) as cleaning solution. The thermal disinfection was carried out at 93°C for 5 min. The used program is based on vario TD (see elsewhere in this document). After cleaning/disinfection the test material was removed from the washer-disinfector under clean conditions. Cleaning and disinfection was performed once.

### Sterilisation

The test materials were packaged in hospital common sterilisation packaging (paper/film bag) and exposed to the steam sterilizer (Systec V-150, Systec Labor Systemtechnik, serial no. 1597) to 121 °C for 20 minutes by use of a fractionated vacuum procedure.

A single sterilization procedure was performed after the cleaning and disinfection cycle.

**Table 5: Extract Preparation**

| Medium | Surface to volume ratio | Temperature | Time |
|---|---|---|---|
| **Cytotoxicity (1x reprocessed)** | | | |
| 1.5% v/v DMSO in DMEM-FBS | 3cm²/mL | 37±1°C | 7 days |

| **Chemical Analysis (organic; 1x reprocessed)** | | | |
|---|---|---|---|
| Ethanol / water | 3cm²/mL | 37±1°C | 7 days |
| Isopropanol | 3cm²/mL | 37±1°C | 24±2h |

**Table 6: Results**

| **Test** | **Result Conclusion** |
|---|---|
| **Cytotoxicity** | |
| *In vitro* cytotoxicity testing on extract of according to ISO 10993-5: 2009 (Biological evaluation of medical devices - Part 5: Tests for *in vitro* cytotoxicity) | Proliferation of L929 cell cultures was not affected in presence of the test item compared to untreated reagent control cultures. |

| **Chemical Analysis** | |
|---|---|
| Chemical analysis - characterization of organic leachables according to ISO 10993-18: 2009 (Biological evaluation of medical devices - Part 18: Chemical characterization of materials) | The test material showed no changes in colour or shape after extraction. The extracts did not optically differ from the blanks. |
| | The ethanol/water and isopropanol extract did not contain detectable organic substances. |

### Body Contact of Device

Because of the permanent contact of the abutment with the gingiva, temporary abutments are classified as surface devices with contact to breached or compromised surfaces and permanent contact duration (>30 days).

### External expert evaluation

Medical Device Services (Lilienthalstrasse 4, 82205 Gilching, Germany) prepared Biological Toxicological Evaluations for the tested devices. It was concluded that the cleaning/disinfection/sterilisation procedure of the final devices does not affect the biological safety of the polycon® ae for the following reasons:
- Extracts of devices were tested in a cell culture system which allows a very sensitive characterization of the resting solubility of materials (organic and inorganic leachable residues and contaminants). Additionally, a prolonged extraction time (7 days, 37°C) was used to intensify the migration of residues. As for the initially performed testing of the semi-finished material no cytotoxic effects have been observed in presence of the extracts.
- The results of highly sensitive chemical analyses (GC-FID. GC-MS) confirm that unchanged inertness of the polymeric material. The total amount of leachables was below the detection limit of 1 mg/cm²/7 days under simulated use conditions (water/ethanol extracts, 7 days, 37 DC). As well, only residues of aliphatic hydrocarbons have been identified in solvent extracts prepared under strongly exaggerated conditions (isopropanol. 24 h, 37°C).
- The non-performance of additional toxicological tests (e.g. systemic toxicity, tissue/mucosa irritation, sensitization, genotoxicity/carcinogenicity, chronic toxicity) with extracts of the device is justified because of the chemically defined composition and the unchanged insoluble properties of the polymeric component substantiated by highly sensitive biological and chemical tests (characterization of leachable/extractable profile).

### Conclusion

Based on the available biocompatibility test data and the external expert evaluation it was concluded that the steam sterilised PMMA components containing polycon® ae are safe for their intended use as dental secondary components.

### Example 4: Biocompatibility Test of Temporary Abutments made from PMMA (Vita® CAD-Temp®).

**Material used:** The temporary abutments made from PMMA (Vita® CAD-Temp®) and according to the same specifications as Example 2.

Testing was performed at Medical Device Service (address as above); an accredited testing laboratory. The studies have been conducted according to the Good Laboratory Practice (GLP) requirements.

### Cleaning and Disinfection

Test material was placed in a cleaning tray positioned in a washer-disinfector (disinfector G 7836 CD, Miele, Gütersloh). The automated cleaning was carried out using neodisher® MediClean (Dr Weigert) as the cleaning solution. The thermal disinfection was carried out at 93°C for 5 min. The used program is based on vario TD (see elsewhere in this document). After cleaning/disinfection the test material was removed from the washer-disinfector under clean conditions.

### Sterilisation

The test materials were packaged in hospital common sterilisation packaging (paper/film bag) and exposed in the steam sterilizer (Systec V-150, Systec LaborSystemtechnik, serial no. 1597).
Sterilisation was performed at two different temperatures:
- 121 °C for 20 minutes
- 134°C for 30 minutes
by use of a fractionated vacuum procedure.

**Table 7: Extract Preparation**

| | **Surface to volume ratio** | **Temperature** | **Time** |
|---|---|---|---|
| **Cytotoxicity** | | | |
| 1.5% v/v DMSO in DMEM-FBS | 3cm²/mL | 37±1°C | 7 days |

| **Chemical Analysis** | | | |
|---|---|---|---|
| 5% Ethanol in distilled water | 3cm²/mL | 37±1°C | 7 days |
| Isopropanol | 3cm²/mL | 37±1°C | 24 hours |

**Table 8: Results**

| **Test** | **Result Conclusion** |
|---|---|
| **Sterilisation Temperature 121°C** | |
| **Cytotoxicity** | |
| *In vitro* cytotoxicity testing on extract of according to ISO 10993-5: 2009 (Biological evaluation of medical devices - Part 5: Tests for *in vitro* cytotoxicity) | The results indicate that the cleaned, disinfected and steam exposed test material does not release substances in cytotoxic concentrations |

| **Chemical Analysis** | |
|---|---|
| Chemical analysis - characterization of organic leachables according to ISO 10993-18: 2009 (Biological evaluation of medical devices - Part 18: Chemical characterization of materials) | The amount of organic substances in the ethanol/water extract was below the limit of quantification. |

| **Sterilisation Temperature 134°C** | |
|---|---|
| The isopropanol extract did not contain detectable organic substances. | |
| **Cytotoxicity** | |
| *In vitro* cytotoxicity testing on extract of according to ISO 10993-5: 2009 (Biological evaluation of medical devices - Part 5: Tests for *in vitro* cytotoxicity) | The results indicate that the cleaned, disinfected and steam exposed test material does not release substances in cytotoxic concentrations |

| **Chemical Analysis** | |
|---|---|
| Chemical analysis - characterization of organic leachables according to ISO 10993-18: 2009 (Biological evaluation of medical devices - Part 18: Chemical characterization of materials) | The amount of organic substances in the ethanol/water extract was below the limit of quantification. |
| | The isopropanol extract did not contain detectable organic substances. |

### Body Contact of Device

Because of the permanent contact of the abutment with the gingiva (intact and compromised) the temporary abutments were classified as surface devices with contact to breached or compromised surfaces and permanent contact duration (>30 days) according to ISO 10993-1.

### External expert evaluation

Medical Device Services prepared a biological safety - toxicology evaluation and they concluded that temporary abutments made from PMMA (Vita® CAD-Temp®) are in compliance with ISO 10993-1 for the dental use. There is no evidence that effects hazardous to the patient will arise by leachable ingredients and/or residues.

### Conclusion

Based on the available biocompatibility test data and the biological safety - toxicology evaluation it was concluded that the temporary abutments made from PMMA (Vita® CAD-Temp®) are safe for its intended use and product safety is ensured even after steam sterilisation of the PMMA components.

### Other embodiments

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

Unless expressly described to the contrary, each of the preferred features described herein can be used in combination with any and all of the other herein described preferred features.

### References

1. "Guideline for Disinfection and Sterilisation in Healthcare Facilities, 2008", US Centers for Disease Control and Prevention, and "Bundesgesundheitsbl-Gesundheitsforsch-Gesundheitsschutz 11.2001" Robert Koch Institut, Germany.

## Claims

1. A method of sterilising a dental secondary component at least partially formed from thermoset poly(methyl methacrylate) (PMMA) comprising the step of;
subjecting the dental secondary component to steam sterilisation.

2. The method according to claim 1, wherein said steam sterilisation is carried out at a temperature between 121 and 134°C for a time of up to 30 minutes.

3. The method according to claim 1 or 2, wherein said steam sterilisation is carried out at 121°C for at least 15 min, preferably 20-25 min.

4. The method according to claim 1 or 2, wherein said steam sterilisation is carried out at between 132-134°C for at least 3 min, preferably 3-6 min.

5. The method according to any one of the preceding claims, wherein the thermoset PMMA is cross-linked by a multifunctional methacrylate molecule and comprises at least 75 wt% methylmethacrylate and up to 25 wt% multimethacrylate cross-linker.

6. The method according to any one of the preceding claims, wherein the thermoset PMMA has a softening temperature of between 105-120°C.

7. The method according to any one of the preceding claims, wherein the dental secondary component is suitable for use in single tooth restorations.

8. The method according to any one of the preceding claims, wherein the thermoset PMMA has a flexural strength of at least 80 MPa, preferably between 80 and 150 MPa.

9. The method according to any one of the preceding claims, wherein the thermoset PMMA has a flexural modulus of at least 2500 MPa, preferably between 2500-3000 MPa.

10. The method according to any one of the preceding claims, wherein the thermoset PMMA has an elastic modulus of at least 2500 MPa, preferably between 2500-3000 MPa.

11. The method according to any one of the preceding claims further comprising the step of cleaning said component with one or more cleaning agents, said cleaning being carried out before said steam sterilisation.

12. The method according to any one of the preceding claims, further comprising the step of disinfecting said component, said disinfecting step being carried out before said steam sterilisation.

13. The method according to any one of the preceding claims, wherein said component is an abutment for a dental implant or a gingiva former.

14. The method according to claim 13, wherein said component comprises an inlay formed of a harder material than the thermoset PMMA and having connection geometry at its apical end for direct connection to a dental implant.

15. A sealed, non-sterile container comprising an unsterile dental secondary component at least partially formed from thermoset PMMA for sterilisation by steam sterilisation.

16. A kit comprising one or more dental secondary component(s) at least partially formed from thermoset PMMA and one or more tray(s) for housing said component(s) during steam sterilisation.
